# EUROPEAN PATENT SPECIFICATION

(11) **EP 3 897 535 B1**
(45) Date of publication and mention of the grant of the patent: **09.12.2026**
(21) Application number: 19832960.9
(22) Date of filing: 20.12.2019
(51) Int. Cl.: A61K 8/31, A61K 8/85, A61Q 1/06

(54) **COSMETIC COMPOSITION COMPRISING A POLYHYDROXYALKANOATE IN AN OILY MEDIUM**
KOSMETISCHE ZUSAMMENSETZUNG ENTHALTEND EIN POLYHYDROXYALKANOAT IN EINEM ÖLIGEN MEDIUM
COMPOSITION COSMÉTIQUE COMPRENANT UN POLYHYDROXYALCANOATE DANS UN MILIEU HUILEUX

(30) Priority: 20.12.2018 FR 1873652
(43) Date of publication of application: 27.10.2021
(73) Proprietor: L'OREAL, 75008 Paris (FR)
(72) Inventor: PORTAL, Julien, 93601 AULNAY-SOUS-BOIS (FR); GARCON, Romain, 93601 AULNAY-SOUS-BOIS (FR)
(74) Representative: L'Oreal
(86) International application number: PCT/EP2019/086809
(87) International publication number: WO 2020/128050

(56) References cited:
- EP-A1- 1 980 235
- WO-A1-2015/123049
- WO-A1-2018/178899

## Description

The present invention relates to a cosmetic composition comprising a polyhydroxyalkanoate in an oily medium, as well as to a method for the treatment of keratinous substances employing such a composition.

It is known to use, in cosmetics, film-forming polymers which can be transported in organic media, such as hydrocarbon oils. Polymers are used in particular as film-forming agent in makeup products, such as mascaras, eyeliners, eye shadows or lipsticks.

The document FR-A-2964663 describes a cosmetic composition comprising pigments coated with a C₃-C₂₁ polyhydroxyalkanoate, such as poly(hydroxybutyrate-co-hydroxyvalerate).

The document WO2011/154508 describes a cosmetic composition comprising a 4-carboxy-2-pyrrolidinone ester derivative and a film-forming polymer which can be a polyhydroxyalkanoate, such as polyhydroxybutyrate, polyhydroxyvalerate and polyhydroxybutyrate-co-polyhydroxyvalerate.

The document US-A-2015/274972 describes a cosmetic composition comprising a cosmetic composition comprising a thermoplastic resin, such as a polyhydroxyalkanoate, in aqueous dispersion and a silicone elastomer.

The document WO 2018/17899 discloses a cosmetic composition comprising a polyhydroxyalkanoate (PHA) in the form of solid particles and an oily phase. PHA is used to absorb oily substance in order to obtain a "soft focus" effect on skin.

A need thus exists to have available a composition comprising a solubilized polyhydroxyalkanoate making it possible to obtain a film exhibiting good cosmetic properties, in particular good resistance to oils and to sebum, as well as a good mattness.

The applicant company has discovered that specific polyhydroxyalkanoate copolymers, as defined below, can be easily employed in the oily media specially selected, thus making it possible to obtain homogeneous compositions because of the good solubilization of the copolymer in the oily medium. The composition exhibits good stability, in particular after storage for one month at ambient temperature (25°C). The composition, in particular after its application to keratinous substances, makes it possible to obtain a film having good cosmetic properties, in particular good resistance to oils and to sebum, as well as a matt appearance.

A subject matter of the present invention is thus a composition comprising:
a) a polyhydroxyalkanoate copolymer comprising, preferably consisting of, the following polymeric units A, B and C:

   -[-O-CH(R1)-CH₂-CO-]- unit A

   -[-O-CH(R2)-CH₂-CO-]- unit B

   -[-O-CH(R3)-CH₂-CO-]- unit C

   in which:
   ■ R1 denotes a linear alkyl radical having from 5 to 9 carbon atoms;
   ■ R2 denotes a linear alkyl radical having a carbon number corresponding to the number of carbon atoms of the R1 radical - 2;
   ■ R3 denotes a linear alkyl radical having a carbon number corresponding to the number of carbon atoms of the R1 radical - 4;
   the molar percentage of unit A being greater than the molar percentage of unit B and greater than the molar percentage of unit C;
b) an oily medium comprising a non-silicone oil chosen from:
   ■ ester oils, carbonate oils,
   ■ branched nonpolar hydrocarbon oils having from 8 to 14 carbon atoms, as a mixture with a monoalcohol having from 2 to 6 carbon atoms according to a monoalcohol/branched nonpolar hydrocarbon oil ratio by weight ranging from 1/99 to 10/90; and

   when the polymer is such that the R1 alkyl group comprises from 6 to 9 carbon atoms, the non-silicone oil is also chosen from nonpolar hydrocarbon oils having from 8 to 14 carbon atoms in the absence of monoalcohol having from 2 to 6 carbon atoms;
   when the polymer is such that the R1 alkyl group comprises 9 carbon atoms, the non-silicone oil is also chosen from hydrogenated polyisobutylenes.

Another subject matter of the invention is a nontherapeutic cosmetic method for the treatment of keratinous substances, comprising the application, to the keratinous substances, of a composition as defined above. The treatment method is in particular a method for caring for or making up keratinous substances.

The polyhydroxyalkanoate copolymer of the composition according to the invention comprises the units A, B and C as defined above.

Preferably, in the copolymer, the unit A is present in a molar percentage ranging from 40% to 97.5%, the unit B is present in a molar percentage ranging from 2% to 40% and the unit C is present in a molar percentage ranging from 0.5% to 20%, with respect to all of the units A, B and C.

According to a first embodiment of the composition according to the invention, the copolymer comprises units A having an R1 alkyl radical comprising 5 carbon atoms, units B having an alkyl radical having 3 carbon atoms and units C having an alkyl radical having 1 carbon atom. Advantageously, the copolymer comprises from 85 mol% to 97.5 mol% of unit A, from 2 mol% to 10 mol% of unit B and from 0.5 mol% to 7 mol% of unit C.

According to a second embodiment of the composition according to the invention, the copolymer comprises units A having an R1 alkyl radical comprising 6 carbon atoms, units B having an alkyl radical having 4 carbon atoms and units C having an alkyl radical having 2 carbon atoms. Advantageously, the copolymer comprises from 60 mol% to 94.5 mol% of unit A, from 5 mol% to 35 mol% of unit B and from 0.5 mol% to 7 mol% of unit C.

According to a third embodiment of the composition according to the invention, the copolymer comprises units A having an R1 alkyl radical comprising 9 carbon atoms, units B having an alkyl radical having 7 carbon atoms and units C having an alkyl radical having 5 carbon atoms. Advantageously, the copolymer comprises from 40 mol% to 50 mol% of unit A, from 30 mol% to 40 mol% of unit B and from 10 mol% to 20 mol% of unit C.

The copolymer preferably has a number-average molecular weight ranging from 50 000 to 150 000.

The molecular weight can be measured in particular by size exclusion chromatography. A method is described below in the examples.

The copolymer can be present in the composition according to the invention in a content ranging from 0.1% to 30% by weight, with respect to the total weight of the composition, preferably ranging from 0.1% to 25% by weight.

Advantageously, the composition can comprise the non-silicone oil of the oily medium in a content ranging from 2% to 99.9% by weight, with respect to the total weight of the composition, preferably ranging from 5% to 90% by weight, preferably ranging from 10% to 80% by weight, preferably ranging from 20% to 80% by weight.

The copolymer can be obtained in a known way by biosynthesis, for example with the microorganisms belonging to the genus *Pseudomonas,* such as *Pseudomonas resinovorans, Pseudomomonas putida, Pseudomonas fluorescens, Pseudomonas aeruginosa, Pseudomonas citronellolis, Pseudomonas mendocina, Pseudomonas chlororaphis* and preferably *Pseudomonas putida;* and with a carbon source which can be a C₂-C₂₀, preferably C₆-C₁₈, carboxylic acid, such as acetic acid, propionic acid, butyric acid, hexanoic acid, heptanoic acid, octanoic acid, nonanoic acid, dodecanoic acid; a saccharide, such as fructose, maltose, lactose, xylose, arabinose, and the like); an n-alkane, such as hexane, octane or dodecane; an n-alcohol, such as methanol, ethanol, octanol or glycerol; methane or carbon dioxide.

The biosynthesis can optionally be carried out in the presence of an inhibitor of the β-oxidation pathway, such as acrylic acid, methacrylic acid, propionic acid, cinnamic acid, salicylic acid, pentenoic acid, 2-butynoic acid, 2-octynoic acid or phenylpropionic acid, and preferably acrylic acid.

Nutrients, such as water-soluble salts based on nitrogen, phosphorus, sulfur, magnesium, sodium, potassium and iron, can also be used for the biosynthesis.

The appropriate known conditions of temperature, pH and dissolved oxygen (DO) can be used for the culturing of the microorganisms.

The microorganisms can be cultured according to any known method of culturing, such as in an unfed or fed mode, batch, continuous mode bioreactor.

The biosynthesis of the polymers used according to the invention is described in particular in the paper "Biosynthesis and Properties of Medium-Chain-Length Polyhydroxyalkanoates with Enriched Content of the Dominant Monomer", Xun Juan et al., Biomacromolecules, 2012, 13, 2926-2932, and in the application WO2011/069244.

The oily medium of the composition according to the invention comprises a non-silicone oil as described above.

The term "oil" is understood to mean a water-immiscible non-aqueous compound which is liquid at ambient temperature (25°C) and atmospheric pressure (760 mmHg).

The term "hydrocarbon oil" is understood to mean an oil consisting of carbon and hydrogen atoms.

The hydrocarbon oil having from 8 to 16 carbon atoms can be chosen from:
■ branched C₈-C₁₆ alkanes, such as C₈-C₁₆ isoalkanes of petroleum origin (also known as isoparaffins), such as isododecane (also known as 2,2,4,4,6-pentamethylheptane), isodecane, isohexadecane and, for example, the oils sold under the Isopar or Permethyl trade names,
■ linear C₈-C₁₆ alkanes, such as n-dodecane (C₁₂) and n-tetradecane (C₁₄), sold by Sasol respectively under the references Parafol 12-97 and Parafol 14-97, and also their mixtures, the undecane/tridecane mixture, the mixtures of n-undecane (C₁₁) and of n-tridecane (C₁₃) which are obtained in examples 1 and 2 of the application WO 2008/155059 of Cognis, and their mixtures.

The term "ester oil" is understood to mean an oily compound having one or more ester groups in its chemical structure.
The ester oil can be chosen from:
■ oils of vegetable origin, such as triglycerides consisting of fatty acid esters of glycerol in which the fatty acids can have varied chain lengths from C₄ to C₂₄, it being possible for the latter to be saturated or unsaturated and linear or branched; these oils are in particular triglycerides of heptanoic acid or octanoic acid. The oils of vegetable origin can be chosen from wheat germ, sunflower, grape seed, sesame, peanut, corn, apricot, castor, shea, avocado, olive, soybean, sweet almond, palm, rapeseed, cottonseed, coconut, hazelnut, walnut, rice, linseed, macadamia, alfalfa, poppy, pumpkinseed, sesame, squash, rapeseed, blackcurrant, evening primrose, millet, barley, quinoa, rye, safflower, candlenut, passionflower, muscat rose or argan oil; shea butter; or also triglycerides of caprylic/capric acids, such as those sold by Stéarineries Dubois or those sold under the names Miglyol 810^{®}, 812^{®} and 818^{®} by Dynamit Nobel,
■ monoester oils of formula R₄COOR₅ in which R₄ represents a hydrocarbon chain comprising from 5 to 19 carbon atoms and R₅ represents a hydrocarbon chain, in particular a branched hydrocarbon chain, containing from 4 to 20 carbon atoms, provided that R₄ + R₅ is ≥ 9 carbon atoms and preferably < 29 carbon atoms, such as, for example, cetearyl octanoate (PurCellin oil), isopropyl myristate, isopropyl palmitate, hexyl laurate, isononyl isononanoate, 2-ethylhexyl palmitate, isostearyl isostearate, 2-hexyldecyl laurate, 2-octyldecyl palmitate, 2-octyldodecyl myristate, 2-ethylhexyl hexanoate, isononyl hexanoate, neopentyl hexanoate, caprylyl heptanoate or octyl octanoate,
■ esters of lactic acid and of C₁₀₋₂₀ alcohol, such as isostearyl lactate, 2-octyldodecyl lactate, myristyl lactate, C₁₂-C₁₃ alkyl lactate (Cosmacol^{®} Eli from Sasol), cetyl lactate or lauryl lactate,
■ diesters of malic acid and of C₁₀-C₂₀ alcohol, such as diisostearyl malate, di(C₁₂-C₁₃ alkyl) malate (Cosmacol^{®} EMI from Sasol), dibutyloctyl malate, diethylhexyl malate or dioctyldodecyl malate,
■ esters of pentaerythritol and of C₈-C₂₂ carboxylic acid (in particular tetraesters or diesters), such as pentaerythrityl tetraoctanoate, pentaerythrityl tetraisostearate, pentaerythrityl tetrabehenate, pentaerythrityl tetracaprylate/tetracaprate, pentaerythrityl tetracocoate, pentaerythrityl tetraethylhexanoate, pentaerythrityl tetraisononanoate, pentaerythrityl tetrastearate, pentaerythrityl tetraisostearate, pentaerythrityl tetralaurate, pentaerythrityl tetramyristate, pentaerythrityl tetraoleate or pentaerythrityl distearate;
■ diesters of following formula (II) R6-O-C(=O)-R'6-C(=O)-O-R"6, with R6 and R'6, which are identical or different, represent a saturated or unsaturated (preferably saturated) and linear or branched C₄ to C₁₂ and preferably C₅ to C₁₀ alkyl chain optionally exhibiting at least one saturated or unsaturated, preferably saturated, ring, and R'6 represents a saturated or unsaturated C₁ to C₄, preferably C₂ to C₄, alkylene chain, such as, for example, an alkylene chain derived from succinate (in this case R'6 is a saturated C₂ alkylene chain), maleate (in this case R'6 is an unsaturated C₂ alkylene chain), glutarate (in this case R'6 is a saturated C₃ alkylene chain) or adipate (in this case R'6 is a saturated C₄ alkylene chain); in particular, R6 and R"6 are chosen from isobutyl, pentyl, neopentyl, hexyl, heptyl, neoheptyl, 2-ethylhexyl, octyl, nonyl and isononyl; mention may preferentially be made of dicaprylyl maleate or di(2-ethylhexyl) succinate;
■ diesters of following formula (III) R7-C(=O)-O-R'7-O-C(=O)-R"7, with R7 and R"7, which are identical or different, represent a C₄ to C₁₂ alkyl chain represent a saturated or unsaturated (preferably saturated) and linear or branched C₄ to C₁₂ and preferentially C₅ to C₁₀ alkyl chain, and R'7 represents a saturated or unsaturated C₁ to C₄, preferably C₂ to C₄, alkylene chain. Mention may in particular be made of 1,3-propanediol dicaprylate (R7 as C₇ and R'7 as C₃), sold under the name Dub Zenoat by Stéarinierie Dubois, or dipropylene glycol dicaprylate;
■ carbonate oils which can be chosen from the carbonates of formula R8-O-CO-OR9, with R8 and R9, which are identical or different, represent a linear or branched C₄ to C₁₂ and preferentially C₆ to C₁₀ alkyl chain; the carbonate oils can be dicaprylyl carbonate (or dioctyl carbonate), sold under the name Cetiol CC^{®} by BASF, di(2-ethylhexyl) carbonate, sold under the name Tegosoft DEC^{®} by Evonik, dipropylheptyl carbonate (Cetiol 4 All from BASF), dibutyl carbonate, dineopentyl carbonate, dipentyl carbonate, dineoheptyl carbonate, diheptyl carbonate, diisononyl carbonate or dinonyl carbonate and preferably dioctyl carbonate.

As described above, the oily medium can comprise a monoalcohol of 2 to 6 carbon atoms. This monoalcohol can be chosen from ethanol, propanol, butanol, pentanol or hexanol. Preferably, the monoalcohol is ethanol or 1-butanol and preferentially ethanol.

Advantageously, the composition according to the invention comprises a physiologically acceptable medium. In particular, the composition is a cosmetic composition.

The term "physiologically acceptable medium" is understood to mean a medium which is compatible with human keratinous substances, such as, for example, the skin, the lips, the nails, the eyelashes, the eyebrows or the hair.

The term "cosmetic composition" is understood to mean a composition which is compatible with keratinous substances, which exhibits a pleasant color, odor and feel, and which does not generate unacceptable discomfort (tingling, tightness, redness) liable to dissuade the consumer from using it.

The term "keratinous substances" is understood to mean the skin (body, face, outline of the eyes, scalp), head hair, eyelashes, eyebrows, body hairs, nails or lips.

The composition according to the invention can comprise a cosmetic additive chosen from water, fragrances, preservatives, fillers, colorants, UV screening agents, oils, waxes, surfactants, moisturizers, vitamins, ceramides, antioxidants, agents for combating free radicals, polymers and thickeners.

Advantageously, the composition according to the invention is a makeup composition, in particular a lip makeup composition, a mascara, an eyeliner, an eye shadow or a foundation.

The composition according to the invention can be in the form of an anhydrous composition, a water-in-oil emulsion or an oil-in-water emulsion.

The term "anhydrous composition" is understood to mean a composition containing less than 2% by weight of water, indeed even less than 0.5% of water and in particular devoid of water. If appropriate, such small amounts of water can in particular be introduced by ingredients of the composition which may contain residual amounts of it.

The invention is illustrated in more detail in the following examples. The amounts are expressed as percentage by weight.

### Examples

### Example 1:

A polymer was prepared using the *Pseudomonas putida* KT2440 ATCC^{®} 47054^{™} microorganism and octanoic acid.

The culturing method was carried out under batch axenic conditions in 5 l Fernbach flasks (Corning^{®} ref. 431685) containing 2 l of culture medium, shaken at 110 rev/min at 30°C in an orbital incubator (diameter of the orbit of 2.5 cm).

The synthesis process was carried out using two distinct culture media. The first culture medium, defined CM1 "inoculum", was used for the preparation of the inoculum. The second culture medium, defined CM2 "batch", was used for unfed batch growth of the microorganism with octanoic acid in the Fernbach flasks.

The composition in grams per liter of the two media is described in the table below:

| | **CM1 "inoculum"** | **CM2 "batch"** |
|---|---|---|
| (NH₄)₂SO₄ | 4.7 | 5.02 |
| Na₂HPO₄ · 7H₂O | 12 | 2.24 |
| KH₂PO₄ | 2.7 | 0.5 |
| Glucose | 9 | 3.9 |
| MgSO₄ · 7H₂O | 0.8 | 1.03 |
| Citric acid | 1.6 | 1.03 |
| Nutrient Broth (1) | 1 | / |
| Octanoic acid | / | 3.8 |
| Solution Microelements (2) | / | 1.4 |
| 2N NaOH | q.s.for pH=6.8 | |
| Water | q.s.for 1000 g | |

(1) The composition of the Nutrient Broth, as percentage by weight, is 37.5% beef extract and 62.5% peptone. Reference 233000 DIFCO^{™}.
(2) The composition of the solution of microelements in grams per liter is described in the table below:

| | |
|---|---|
| FeSO₄·7H₂O | 10.0 g |
| CaCl₂·2H₂O | 3.0 g |
| ZnSO₄·7H₂O | 2.2 g |
| MnSO₄·4H₂O | 0.5 g |
| H₃BO₃ | 0.3 g |
| CoCl₂·6H₂O | 0.2 g |
| Na₂MoO₄·2H₂O | 0.15 g |
| NiCl₂·6H₂O | 0.02 g |
| CuSO₄·5H₂O | 1.00 g |
| 0.5N HCl | q.s.for 1000 g |

100 ml of inoculum were prepared by suspending a cryotube containing 1 ml of the strain with 100 ml of "inoculum" culture medium at a pH adjusted to 6.8 with 2N NaOH in a 250 ml Fernbach flask and then incubated at 30°C at 150 rev/min for 24 h. 1.9 l of CM2 culture medium "BATCH" placed in a presterilized 5 l Fernbach flask were inoculated at OD = 0.1 with 100 ml of inoculum. After 70 hours at 30°C at 110 rev/min, the biomass was dried by lyophilization before being extracted with dichloromethane for 24 h. The suspension was clarified by filtration on a GF/A filter (Whatman^{®}). The filtrate, containing the copolymer in solution in the dichloromethane, was concentrated by evaporation and then dried under high vacuum at 40°C to constant weight. The crude polyhydroxyalkanoate was purified by precipitation of a solution of the latter in solution in 10 times its weight of dichloromethane from 10 volumes of the solution of cold methanol. The solid obtained was dried under high vacuum at 40°C to constant weight.

The molecular weight of the polyhydroxyalkanoate obtained was characterized by size exclusion chromatography, refractive index detection.
■ Eluent: THF
■ Analytical flow: 1 ml/min
■ Injection: 100 µl
■ Columns: 1 Agilent PLGel Mixed-D column 5 µm; 300 x 7.5 mm; 1 Agilent PLGel Mixed-C column 5 µm; 300 x 7.5 mm; 1 Agilent Oligopore column; 300 x 7.5 mm
■ at ambient temperature (25°C)
■ Detection: Waters 2487 Dual l Absorbance Detector, Waters 2414 Refractive Index Detector
■ Integrator: refractive index at 45°C and 64 mV
■ Empower (GPC Module Relative/conventional molar mass)
■ Injection time Empower 40 min
■ Standards: High-weight polystyrene/EasiVial PS-H 4 ml from Agilent Technology Part No. PL2010-0200

The analysis makes it possible to measure the weight-average molecular weight (Mw in g/mol), the number-average molecular weight (Mn in g/mol), the polydispersity index PI (Mw/Mn) and the degree of polymerization DPn.

The monomeric composition of the polyhydroxyalkanoate obtained was defined by gas chromatography equipped with a flame ionization detector.

The identification is carried out via the injection of commercial standards and the monomeric composition was determined by methanolysis and silylation treatment.

In order to determine the monomeric composition, 7 mg of the polyhydroxyalkanoate polymer were dissolved in 1.5 ml of chloroform and were methanolyzed in the presence of 1.5 ml of a MeOH/HCI (17/2, v/v) solution at 100°C for 4 h. The organic phase was subsequently washed with 1 ml of water and then dried over MgSO₄. The silylation of the methyl esters formed was carried out by adding 100 µl of BSTFA (N,O-bis(trimethylsilyl)trifluoroacetamide) and 100 µl of pyridine to the methylated sample. The solution was heated at 70°C for 1 h and then evaporated to dryness. The sample was then dissolved in 600 µl of dichloromethane and analyzed by chromatography under the following conditions:
■ Hewlett Packard 6890 Series appliance
■ Stationary phase column ZB-5 HT from Phenomenex (length: 30 m, diameter: 0.25 mm)
■ Temperature: isothermal 60°C to 300°C in 6 min (heating rate: 10°C/min)
■ Gas: Helium; flow rate: 0.8 ml/min
■ Injector: Temperature: 250°C; 50 ml/min
■ Flame ionization detector; Temperature: 300°C
■ Injection: Volume 1 µl

A copolymer containing 91% by weight of poly(3-hydroxyoctanoate), 6% by weight of poly(3-hydroxyhexanoate) and 3% by weight of poly(3-hydroxybutanoate) was thus obtained.
■ Mn = 68 100 g/mol
■ Mw = 149 100 g/mol
■ PI = 2.2
■ DPn = 531

### Example 2

A polymer was prepared using the *Pseudomonas putida* KT2440 ATCC^{®} 47054^{™} microorganism, octanoic acid and acrylic acid.

The culturing method was carried out under continuous axenic conditions at a dilution of D = 0.25 h⁻¹ in a 3 l chemostat containing 1.1 l of culture medium. The system was aerated with air at a flow rate of 3 vvm (vvm = volume of air per volume of fermentation medium per minute) for a dissolved oxygen (DO) setpoint at 30% saturation.

The procurement process was carried out using three distinct culture media. The first undefined culture medium (CM1) was used for the preparation of the inoculum. The second defined culture medium (CM2) was used for the unfed batch growth of the microorganism in the fermenter. The third defined culture medium (CM3) was used for the feeding, or maintenance, of the continuous fermentation containing octanoic acid and acrylic acid (inhibitor of the β-oxidation pathway).

The CM1 and CM2 media are identical to those described in example 1. The composition in grams per liter of the CM3 medium is described in the table below:

| | CM3 "cotinuous" |
|---|---|
| (NH₄)₂SO₄ | 502 |
| Na₂HPO₄ · 7H₂O | 2.24 |
| KH₂PO₄ | 0.5 |
| Glucose | 3 |
| MgSO₄ · 7H₂O | 1.03 |
| Citric acid | 1.03 |
| Nutrient Broth (1) | / |
| Octanoic acid | 3.8 |
| Solution Microelements (2) | 1.4 |
| Acrylic acid | 0.2 |
| 2N NaOH | q.s. for pH=6.8 |
| Water | qs for 1000 g |

100 ml of inoculum were prepared by suspending a cryotube containing 1 ml of the strain with 100 ml of Nutrient Broth at a pH adjusted to 7.0 with 2N NaOH in a 250 ml Fernbach flask and were then incubated at 30°C at 150 rev/min for 24 h.

The fermenter containing 1 liter of CM2 culture medium at 30°C was inoculated at an optical density of 0.1 at 630 nm (OD₆₃₀ = 0.1). The system was maintained at 30°C with stirring of 700 +/- 200 rev/min and adjusted in cascade with oxygenation for approximately 16 h and/or the time that the microorganism can reach its growth plateau.

Feeding the fermenter with the CM3 medium was initiated when the microorganism reached its growth plateau and withdrawal was carried out in order to retain the initial weight of fermentation medium. Once the equilibrium state was reached in continuous culture, a fraction of the withdrawn material was centrifuged in order to separate the biomass from the fermentation medium. The biomass was dried by lyophilization and then extracted with dichloromethane for 24 h. The suspension obtained was clarified by filtration on a GF/A filter (Whatman^{®}). The filtrate obtained, comprising the copolymer in solution in the dichloromethane, was concentrated by evaporation and then dried under high vacuum at 40°C to constant weight. The crude polyhydroxyalkanoate was purified by precipitation of a solution of the latter in solution in 10 times its weight of dichloromethane from 10 volumes of the solution of cold methanol. The solid obtained was dried under high vacuum at 40°C to constant weight.

A copolymer comprising 96% by weight of poly(3-hydroxyoctanoate), 3% by weight of poly(3-hydroxyhexanoate) and 1% by weight of poly(3-hydroxybutanoate) was thus obtained.
■ Mn = 67 900 g/mol
■ Mw = 142 000 g/mol
■ PI = 2.1
■ DPn = 611

### Example 3:

A polymer was prepared according to the procedure of example 2 using nonanoic acid (instead of octanoic acid).

A copolymer comprising 86% by weight of poly(3-hydroxynonanoate), 9% by weight of poly(3-hydroxyheptanoate) and 5% by weight of poly(3-hydroxypentanoate) was thus obtained.
■ Mn = 65 900 g/mol
■ Mw = 143 600 g/mol
■ PI = 2.2
■ DPn = 531

### Example 4:

A polymer was prepared according to the procedure of example 2 using nonanoic acid (instead of octanoic acid) and without acrylic acid.

A copolymer comprising 68% by weight of poly(3-hydroxynonanoate), 27% by weight of poly(3-hydroxyheptanoate) and 5% by weight of poly(3-hydroxypentanoate) was thus obtained.
■ Mn = 55 800 g/mol
■ Mw = 124 500 g/mol
■ PI = 2.2
■ DPn = 469

### Example 5:

A polymer was prepared according to the procedure of example 2 using dodecanoic acid (instead of octanoic acid).

A copolymer comprising 44% by weight of poly(3-hydroxydodecanoate), 38% by weight of poly(3-hydroxydecanoate) and 18% by weight of poly(3-hydroxyoctanoate) was obtained.
■ Mn = 67 400 g/mol
■ Mw = 129 800 g/mol
■PI = 1.9
■ DPn = 484

### Example 6 (outside the invention):

A copolymer was prepared using the *Pseudomonas resinovorans* ATCC^{®} 14235^{™} microorganism, hexanoic acid and acrylic acid according to the procedure of example 2.

The composition of the solution of microelements which is used, in grams per liter, is described in the table below:

| | |
|---|---|
| ZnSO₄·7H₂O | 0.1 g |
| MnCl₂·4H₂O | 0.03 g |
| H₃BO₃ | 0.3 g |
| CoCl₂·6H₂O | 0.2 g |
| Na₂MoO₄·2H₂O | 0.03 g |
| NiCl₂·6H₂O | 0.02 g |
| CuSO₄·5H₂O | 0.01 g |
| 0.5N HCl | q.s. for 1000 g |

A copolymer comprising 95% by weight of poly(3-hydroxyhexanoate) and 5% by weight of poly(3-hydroxyoctanoate) was thus obtained.
■ Mn = 107 200 g/mol
■ Mw = 219 100 g/mol
■PI = 2
■ DPn = 1227

### Example 7 (outside the invention):

A polymer was prepared according to the procedure of example 2 using heptanoic acid (instead of octanoic acid).

A copolymer comprising 96% by weight of poly(3-hydroxyheptanoate) and 4% by weight of poly(3-hydroxypentanoate) was thus obtained.
■ Mn = 83 600 g/mol
■ Mw = 184 200 g/mol
■ PI = 2.2
■ DPn = 845

### Example 8: Test of solubility in oils

The solubility in various oils, which are described in the table below, of the polymers of examples 1 to 7, as well as 3 commercial polymers A to C described below, was evaluated.

1 g of polymer was introduced into 9 g of oil in a flask, the mixture was heated at 70°C with stirring for 1 h, the flask was then placed at ambient temperature for 24 h and the solubility of the polymer in the mixture was observed with the naked eye.

The test was also carried out with 3 commercial polymers outside the invention:
Polymer A: poly(3-hydroxybutanoate), sold under the reference 363502 Aldrich by Sigma-Aldrich
Polymer B: poly(3-hydroxybutanoate)-co-(3-hydroxyvalerate), the 3-hydroxyvalerate unit being present at 12 mol%, sold under the reference 403121 Aldrich by Sigma-Aldrich
Polymer C: poly(3-hydroxybutanoate)-co-(3-hydroxyvalerate), the 3-hydroxyvalerate unit being present at 8 mol%, sold under the reference 403105 Aldrich by Sigma-Aldrich

The following results were obtained:

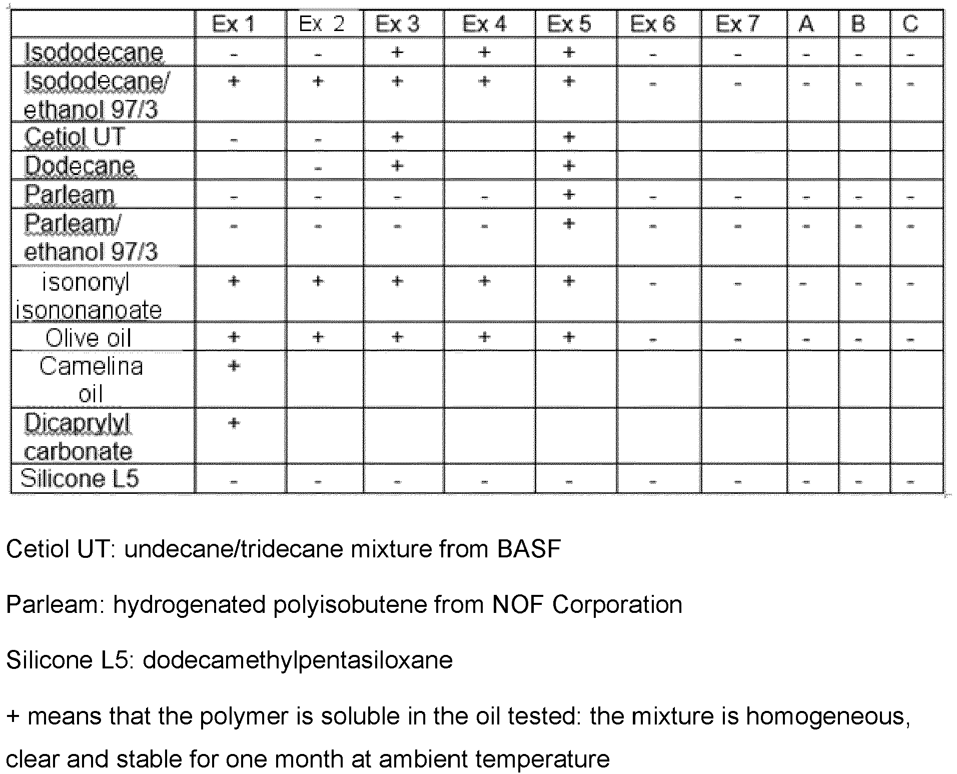

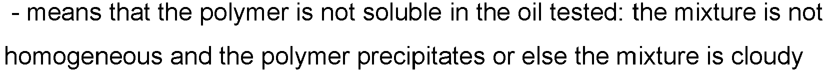

The solubility test for the polymer of example 2 was supplemented with other oils described in the table below. The following results were obtained:

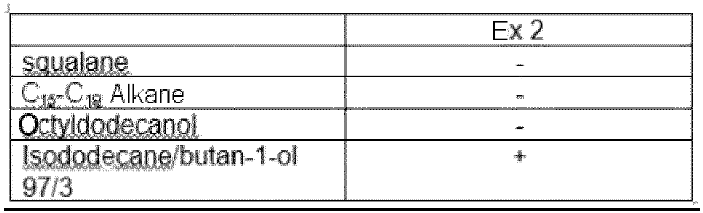

### Example 11: Evaluation of the film-forming and cosmetic properties

A solution of polymer (polymer of the examples at 10% by weight in isododecane) to be evaluated was deposited on a contrast chart (for example that sold under the reference byko-charts by BYK-Gardner) with a film drawer (speed: 50 mm/s - wet deposit 100 µm thick) and the deposited film was dried for 24 hours at ambient temperature (25°C). The dry film has a thickness of approximately 10 µm.

The gloss of the film was measured using a glossmeter (three angles Refo 3/Refo 3D from De Lange, Labomat distributor) on the black part of the contrast chart at an angle of 20°.

The resistance of the film obtained was evaluated by separately depositing, on the dry film, two drops (one drop = 10 µl) of olive oil and two drops of sebum on the black part of the contrast chart. The drops were left in contact with the dry film for 5 minutes and 30 minutes respectively and subsequently the drop of oil was wiped off and the appearance of the region of the film which was in contact with the oil was observed. If the film was damaged by the drop deposited, the polymer film is regarded is being non-resistant to olive oil and/or to sebum.

The tacky aspect of the polymer film was evaluated by touching the dry film with a finger. The following results were obtained:

| Property | **Polymer Example 1** | **Polymer Example 2** | **Polymer Example 3** | **Polymer Example** 4 | **Polymer Example 5** |
|---|---|---|---|---|---|
| **Resistance to Olive oil** | Good resistance | Good resistance | Good resistance | Good resistance | Good resistance |
| **Resistance to sebum** | Good resistance | Good resistance | Good resistance | Good resistance | Good resistance |
| **Gloss** | Matt film | Matt film | Matt film | Matt film | Matt film |
| **Tacky** | Non-tacky | Non-tacky | Non-tacky | Non-tacky | Non-tacky |

The results obtained show that the five polymers according to the invention are film-forming and the film obtained is non-tacky, is matt in appearance and exhibits good resistance to oil and to sebum.

### Example 12: Cosmetic evaluation of makeup compositions

A makeup composition (lipstick) described below was prepared:

| | |
|---|---|
| Polymer of the invention | 20% |
| Pigment paste comprising 40% by weight of pigment DC Red 7 in isododecane | 5% |
| Isododecane | q.s. for 100% |

The composition was prepared with each polymer of examples 1 to 5.

Each composition was applied on a skin equivalent support made of elastomer by producing a deposited layer with a wet thickness of 100 µm, which was left to dry at ambient temperature (25°C) for 24 hours.

The resistance of the film obtained was evaluated by separately applying 0.5 ml of olive oil and 0.5 ml of sebum; after 5 minutes of contact, the surface of the film was rubbed with a cotton swab, 25 passes of the cotton swab over the surface being carried out, and then the state of the film was observed (degraded or undegraded appearance of the film).

The resistance of the film to Scotch tape was also evaluated:
A piece of Scotch tape (Scotch^{®} Magic^{™} 810 from 3M; w = 19 mm, l = 5 cm) was applied to the dry film. A weight of approximately 1070 g (with a surface area of 65 * 55 mm) was placed on the piece of Scotch tape for 30 seconds. The Scotch tape was subsequently removed and subsequently deposited on a microscope slide in order to observe the adherent surface of the Scotch tape applied and to see if it contains traces of the dry polymer film. The following results were obtained:

| Property | **Polymer Example 1** | **Polymer Example 2** | **Polymer Example 3** | **Polymer Example 4** | **Polymer Example 5** |
|---|---|---|---|---|---|
| **Resistance to olive oil** | Good resistance | Good resistance | Good resistance | Good resistance | Good resistance |
| **Resistance to sebum** | Good resistance | Good resistance | Good resistance | Good resistance | Good resistance |
| **Scotch tape test** | Good resistance | Good resistance | Good resistance | Good resistance | Good resistance |

The results obtained show that the compositions according to the invention exhibit good resistance to oil and to sebum and a good persistence (resistance to Scotch tape).

The lipstick composition applied to the lips thus makes it possible to obtain makeup which is resistant to oil and to sebum, thus exhibiting a good persistence.

## Claims

1. A composition comprising:
a) a polyhydroxyalkanoate copolymer comprising, preferably consisting of, the following polymeric units A, B and C:
-[-O-CH(R1)-CH₂-CO-]- unit A
-[-O-CH(R2)-CH₂-CO-]- unit B
-[-O-CH(R3)-CH₂-CO-]- unit C
in which:
■ R1 denotes a linear alkyl radical having from 5 to 9 carbon atoms;
■ R2 denotes a linear alkyl radical having a carbon number corresponding to the number of carbon atoms of the R1 radical - 2;
■ R3 denotes a linear alkyl radical having a carbon number corresponding to the number of carbon atoms of the R1 radical - 4;
the molar percentage of unit A being greater than the molar percentage of unit B and greater than the molar percentage of unit C;
b) an oily medium comprising a non-silicone oil chosen from:
■ ester oils, carbonate oils,
■ branched nonpolar hydrocarbon oils having from 8 to 14 carbon atoms, as a mixture with a monoalcohol having from 2 to 6 carbon atoms according to a monoalcohol/branched nonpolar hydrocarbon oil ratio by weight ranging from 1/99 to 10/90; and
when the polymer is such that the R1 alkyl group comprises from 6 to 9 carbon atoms, the non-silicone oil is also chosen from nonpolar hydrocarbon oils having from 8 to 14 carbon atoms in the absence of monoalcohol having from 2 to 6 carbon atoms;
when the polymer is such that the R1 alkyl group comprises 9 carbon atoms, the non-silicone oil is also chosen from hydrogenated polyisobutylenes.

2. The composition as claimed in claim 1, **characterized in that**, for the copolymer, the unit A is present in a molar percentage ranging from 40% to 97.5%, the unit B is present in a molar percentage ranging from 2% to 40% and the unit C is present in a molar percentage ranging from 0.5% to 20%, with respect to all of the units A, B and C.

3. The composition as claimed in claim 1 or 2, **characterized in that** the copolymer comprises units A having an R1 alkyl radical comprising 5 carbon atoms, units B having an alkyl radical having 3 carbon atoms and units C having an alkyl radical having 1 carbon atom.

4. The composition as claimed in one of claims 1 to 3, **characterized in that** the copolymer comprises from 85 mol% to 97.5 mol% of unit A, from 2 mol% to 10 mol% of unit B and from 0.5 mol% to 7 mol% of unit C.

5. The composition as claimed in claim 1 or 2, **characterized in that** the copolymer comprises units A having an R1 alkyl radical comprising 6 carbon atoms, units B having an alkyl radical having 4 carbon atoms and units C having an alkyl radical having 2 carbon atoms.

6. The composition as claimed in one of claims 1, 2 or 5, **characterized in that** the copolymer comprises from 60 mol% to 94.5 mol% of unit A, from 5 mol% to 35 mol% of unit B and from 0.5 mol% to 7 mol% of unit C.

7. The composition as claimed in either of claims 1 and 2, **characterized in that** the copolymer comprises units A having an R1 alkyl radical comprising 9 carbon atoms, units B having an alkyl radical having 7 carbon atoms and units C having an alkyl radical having 5 carbon atoms.

8. The composition as claimed in one of claims 1, 2 or 7, **characterized in that** the copolymer comprises from 40 mol% to 50 mol% of unit A, from 30 mol% to 40 mol% of unit B and from 10 mol% to 20 mol% of unit C.

9. The composition as claimed in one of the preceding claims, **characterized in that** the copolymer has a number-average molecular weight ranging from 50 000 to 150 000.

10. The composition as claimed in one of the preceding claims, **characterized in that** the copolymer is present in a content ranging from 0.1% to 30% by weight, with respect to the total weight of the composition, preferably ranging from 0.1% to 25% by weight.

11. The composition as claimed in one of the preceding claims, **characterized in that** the nonpolar hydrocarbon oil having from 8 to 16 carbon atoms is chosen from branched C₈-C₁₆ alkanes and linear C₈-C₁₆ alkanes.

12. The composition as claimed in one of claims 1 to 10, **characterized in that** the ester oil is chosen from triglycerides consisting of C₄-C₂₄ fatty acid esters of glycerol; monoester oils of formula R₄COOR₅ in which R₄ represents a hydrocarbon chain comprising from 5 to 19 carbon atoms and R₅ represents a hydrocarbon chain, in particular a branched hydrocarbon chain, containing from 4 to 20 carbon atoms, provided that R₄ + R₅ is ≥ 9 carbon atoms; esters of lactic acid and of C₁₀₋₂₀ alcohol; diesters of malic acid and of C₁₀-C₂₀ alcohol; esters of pentaerythritol and of C₈-C₂₂ carboxylic acid; diesters of formula (II) R6-O-C(=O)-R'6-C(=O)-O-R"6, with R6 and R"6, which are identical or different, represent a saturated or unsaturated and linear or branched C₄ to C₁₂ alkyl chain optionally exhibiting at least one saturated or unsaturated ring, and R'6 represents a saturated or unsaturated C₁ to C₄ alkylene chain; diesters of formula (III) R7-C(=O)-O-R'7-O-C(=O)-R"7, with R7 and R"7, which are identical or different, represent a C₄ to C₁₂ alkyl chain represent a saturated or unsaturated and linear or branched C₄ to C₁₂ alkyl chain, and R'7 represents a saturated or unsaturated C₁ to C₄ alkylene chain.

13. The composition as claimed in one of claims 1 to 10, **characterized in that** the carbonate oils are chosen from the carbonates of formula R8-O-COO-R9, with R8 and R9, which are identical or different, represent a C₄ to C₁₂ alkyl chain.

14. The composition as claimed in one of claims 1 to 10, **characterized in that** it comprises a monoalcohol of 2 to 6 carbon atoms preferably chosen from ethanol or 1-butanol and preferentially ethanol.

15. The composition as claimed in one of the preceding claims, **characterized in that** it comprises a physiologically acceptable medium.

16. A nontherapeutic cosmetic method for the treatment of keratinous substances, comprising the application, to the keratinous substances, of a composition as claimed in one of the preceding claims.

## Patentansprüche

1. Zusammensetzung, umfassend:
a) ein Polyhydroxyalkanoat-Copolymer, vorzugsweise bestehend aus den folgenden Polymereinheiten A, B und C :
-[-O-CH(R1)-CH₂-CO-]- Einheit A
-[-O-CH(R2)-CH₂-CO-]- Einheit B
-[-O-CH(R3)-CH₂-CO-]- Einheit C
wobei:
■ R1 einen linearen Alkylrest mit 5 bis 9 Kohlenstoffatomen bedeutet;
■ R2 einen linearen Alkylrest mit einer Kohlenstoffzahl entsprechend der Anzahl an Kohlenstoffatomen des R1-Restes - 2 bedeutet;
■ R3 einen linearen Alkylrest mit einer Kohlenstoffzahl entsprechend der Anzahl an Kohlenstoffatomen des R1-Restes - 4 bedeutet;
wobei der molare Prozentsatz der Einheit A größer als der molare Prozentsatz der Einheit B und größer als der molare Prozentsatz der Einheit C ist;
b) ein öliges Medium, das ein Nichtsilikonöl umfasst, das ausgewählt ist aus:
▪ Esterölen, Carbonatölen,
▪ verzweigten unpolaren Kohlenwasserstoffölen mit 8 bis 14 Kohlenstoffatomen, im Gemisch mit einem Monoalkohol mit 2 bis 6 Kohlenstoffatomen gemäß einem Gewichtsverhältnis Monoalkohol/verzweigtes unpolares Kohlenwasserstofföl im Bereich von 1/99 bis 10/90; und
wenn das Polymer derartig ist, dass die R1-Alkylgruppe 6 bis 9 Kohlenstoffatome umfasst, das Nichtsilikonöl auch ausgewählt ist aus unpolaren Kohlenwasserstoffölen mit 8 bis 14 Kohlenstoffatomen in Abwesenheit von Monoalkohol mit 2 bis 6 Kohlenstoffatomen;
wenn das Polymer derartig ist, dass die R1-Alkylgruppe 9 Kohlenstoffatome umfasst, das Nichtsilikonöl auch ausgewählt ist aus hydrierten Polyisobutylenen.

2. Zusammensetzung nach Anspruch 1, **dadurch gekennzeichnet, dass** für das Copolymer die Einheit A bei einem molaren Prozentsatz im Bereich von 40 % bis 97,5% vorliegt, die Einheit B bei einem molaren Prozentsatz im Bereich von 2 % bis 40 % vorliegt und die Einheit C bei einem molaren Prozentsatz im Bereich von 0,5 % bis 20% vorliegt, bezogen auf alle der Einheiten A, B und C.

3. Zusammensetzung nach Anspruch 1 oder 2, **dadurch gekennzeichnet, dass** das Copolymer Einheiten A mit einem R1-Alkylrest mit 5 Kohlenstoffatomen, Einheiten B mit einem Alkylrest mit 3 Kohlenstoffatomen und Einheiten C mit einem Alkylrest mit 1 Kohlenstoffatom umfasst.

4. Zusammensetzung nach einem der Ansprüche 1 bis 3, **dadurch gekennzeichnet, dass** das Copolymer 85 Mol-% bis 97,5 Mol-% der Einheit A, 2 Mol-% bis 10 Mol-% der Einheit B und 0,5 Mol-% bis 7 Mol-% der Einheit C umfasst.

5. Zusammensetzung nach Anspruch 1 oder 2, **dadurch gekennzeichnet, dass** das Copolymer Einheiten A mit einem R1-Alkylrest mit 6 Kohlenstoffatomen, Einheiten B mit einem Alkylrest mit 4 Kohlenstoffatomen und Einheiten C mit einem Alkylrest mit 2 Kohlenstoffatomen umfasst.

6. Zusammensetzung nach einem der Ansprüche 1, 2 oder 5, **dadurch gekennzeichnet, dass** das Copolymer 60 Mol-% bis 94, 5 Mol-% der Einheit A, 5 Mol-% bis 35 Mol-% der Einheit B und 0,5 Mol-% bis 7 Mol-% der Einheit C umfasst.

7. Zusammensetzung nach einem der Ansprüche 1 oder 2, **dadurch gekennzeichnet, dass** das Copolymer Einheiten A mit einem R1-Alkylrest mit 9 Kohlenstoffatomen, Einheiten B mit einem Alkylrest mit 7 Kohlenstoffatomen und Einheiten C mit einem Alkylrest mit 5 Kohlenstoffatomen umfasst.

8. Zusammensetzung nach einem der Ansprüche 1, 2 oder 7, **dadurch gekennzeichnet, dass** das Copolymer 40 Mol-% bis 50 Mol-% der Einheit A, 30 Mol-% bis 40 Mol-% der Einheit B und 10 Mol-% bis 20 Mol-% der Einheit C umfasst.

9. Zusammensetzung nach einem der vorhergehenden Ansprüche, **dadurch gekennzeichnet, dass** das Copolymer ein zahlenmittleres Molekulargewicht im Bereich von 50 000 bis 150 000 aufweist.

10. Zusammensetzung nach einem der vorhergehenden Ansprüche, **dadurch gekennzeichnet, dass** das Copolymer in einem Gehalt im Bereich von 0,1 Gew.-% bis 30 Gew.-%, bezogen auf das Gesamtgewicht der Zusammensetzung, vorzugsweise im Bereich von 0,1 Gew.-% bis 25 Gew.-% vorliegt.

11. Zusammensetzung nach einem der vorhergehenden Ansprüche, **dadurch gekennzeichnet, dass** das unpolare Kohlenwasserstofföl mit 8 bis 16 Kohlenstoffatomen ausgewählt ist aus verzweigten C8-C16-Alkanen und linearen C8-C16-Alkanen.

12. Zusammensetzung nach einem der Ansprüche 1 bis 10, **dadurch gekennzeichnet, dass** das Esteröl ausgewählt ist aus Triglyceriden bestehend aus C4-C24-Fettsäureestern von Glycerin; Monoesterölen der Formel R4COOR5, worin R4 für eine Kohlenwasserstoffkette mit 5 bis 19 Kohlenstoffatomen steht und R5 für eine Kohlenwasserstoffkette, insbesondere eine verzweigte Kohlenwasserstoffkette mit 4 bis 20 Kohlenstoffatomen steht, mit der Maßgabe, dass R4 + R5 ≥ 9 Kohlenstoffatome ist; Estern von Milchsäure und C10-20-Alkohol; Diestern von Äpfelsäure und C10-C20-Alkohol; Estern von Pentaerythritol und C8-C22-Carbonsäure; Diestern der Formel (II) R6-O-C(=O)-R'6-C(=O)-O-R"6, wobei R6 und R"6, die gleich oder verschieden sind, für eine gesättigte oder ungesättigte und lineare oder verzweigte C4- bis C12-Alkylkette stehen, die optional mindestens einen gesättigten oder ungesättigten Ring aufweist, und R'6 für eine gesättigte oder ungesättigte C1- bis C4-Alkylenkette steht; Diestern der Formel (III) R7-C(=O-O-R7-O-C(=O)-R"7, wobei R7 und R"7, die gleich oder verschieden sind, für eine C4- bis C12-Alkylkette stehen, für eine gesättigte oder ungesättigte und lineare oder verzweigte C4- bis C12-Alkylkette stehen, und R'7 für eine gesättigte oder ungesättigte C1- bis C4-Alkylenkette steht.

13. Zusammensetzung nach einem der Ansprüche 1 bis 10, **dadurch gekennzeichnet, dass** die Carbonatöle ausgewählt sind aus Carbonaten der Formel R8-O-CO-O-R9, wobei R8 und R9, die gleich oder verschieden sind, für eine C4- bis C12-Alkylkette stehen.

14. Zusammensetzung nach einem der Ansprüche 1 bis 10, **dadurch gekennzeichnet, dass** sie einen Monoalkohol mit 2 bis 6 Kohlenstoffatomen, vorzugsweise ausgewählt aus Ethanol oder 1-Butanol, und bevorzugt Ethanol, enthält.

15. Zusammensetzung nach einem der vorhergehenden Ansprüche, **dadurch gekennzeichnet, dass** sie ein physiologisch akzeptables Medium umfasst.

16. Nicht-therapeutisches kosmetisches Verfahren zur Behandlung keratinischer Substanzen, umfassend das Anwenden einer Zusammensetzung nach einem der vorhergehenden Ansprüche auf die keratinischen Substanzen.

## Revendications

1. Composition comprenant :
a) un copolymère polyhydroxyalcanoate comprenant les unités polymères A, B et C suivantes, de préférence constitué de celles-ci :
-[-O-CH(R1)-CH₂-CO-]- unité A
-[-O-CH(R2)-CH₂-CO-]- unité B
-[-O-CH(R3)-CH₂-CO-]- unité C
dans laquelle :
■ R1 désigne un radical alkyle linéaire ayant de 5 à 9 atomes de carbone ;
■ R2 désigne un radical alkyle linéaire ayant un nombre d'atomes de carbone correspondant au nombre d'atomes de carbone du radical R1 - 2 ;
■ R3 désigne un radical alkyle linéaire ayant un nombre d'atomes de carbone correspondant au nombre d'atomes de carbone du radical R1 - 4 ;
le pourcentage molaire de l'unité A étant supérieur au pourcentage molaire de l'unité B et supérieur au pourcentage molaire de l'unité C ;
b) un milieu huileux comprenant une huile non siliconée choisie parmi :
■ les huiles d'esters, les huiles de carbonates,
■ les huiles d'hydrocarbures ramifiés non polaires ayant de 8 à 14 atomes de carbone, sous forme d'un mélange avec un monoalcool ayant de 2 à 6 atomes de carbone selon un rapport en poids monoalcool/huile d'hydrocarbures ramifiés non polaires se situant dans la plage allant de 1/99 à 10/90 ; et
lorsque le polymère est tel que le groupe alkyle R1 comprend de 6 à 9 atomes de carbone, l'huile non siliconée est également choisie parmi les huiles d'hydrocarbures non polaires ayant de 8 à 14 atomes de carbone en l'absence de monoalcool ayant de 2 à 6 atomes de carbone ;
lorsque le polymère est tel que le groupe alkyle R1 comprend 9 atomes de carbone, l'huile non siliconée est également choisie parmi les polyisobutylènes hydrogénés.

2. Composition selon la revendication 1, **caractérisée en ce que**, pour le copolymère, l'unité A est présente en un pourcentage molaire se situant dans la plage allant de 40 % à 97,5 %, l'unité B est présente en un pourcentage molaire se situant dans la plage allant de 2 % à 40 % et l'unité C est présente en un pourcentage molaire se situant dans la plage allant de 0,5 % à 20 %, par rapport à la totalité des unités A, B et C.

3. Composition selon la revendication 1 ou 2, **caractérisée en ce que** le copolymère comprend des unités A ayant un radical alkyle R1 comprenant 5 atomes de carbone, des unités B ayant un radical alkyle ayant 3 atomes de carbone et des unités C ayant un radical alkyle ayant 1 atome de carbone.

4. Composition selon l'une des revendications 1 à 3, **caractérisée en ce que** le copolymère comprend de 85 % en mole à 97,5 % en mole d'unité A, de 2 % en mole à 10 % en mole d'unité B et de 0,5 % en mole à 7 % en mole d'unité C.

5. Composition selon la revendication 1 ou 2, **caractérisée en ce que** le copolymère comprend des unités A ayant un radical alkyle R1 comprenant 6 atomes de carbone, des unités B ayant un radical alkyle ayant 4 atomes de carbone et des unités C ayant un radical alkyle ayant 2 atomes de carbone.

6. Composition selon l'une des revendications 1, 2 ou 5, **caractérisée en ce que** le copolymère comprend de 60 % en mole à 94,5 % en mole d'unité A, de 5 % en mole à 35 % en mole d'unité B et de 0,5 % en mole à 7 % en mole d'unité C.

7. Composition selon l'une ou l'autre des revendications 1 et 2, **caractérisée en ce que** le copolymère comprend des unités A ayant un radical alkyle R1 comprenant 9 atomes de carbone, des unités B ayant un radical alkyle ayant 7 atomes de carbone et des unités C ayant un radical alkyle ayant 5 atomes de carbone.

8. Composition selon l'une des revendications 1, 2 ou 7, **caractérisée en ce que** le copolymère comprend de 40 % en mole à 50 % en mole d'unité A, de 30 % en mole à 40 % en mole d'unité B et de 10 % en mole à 20 % en mole d'unité C.

9. Composition selon l'une des revendications précédentes, **caractérisée en ce que** le copolymère a une masse moléculaire moyenne en nombre se situant dans la plage allant de 50 000 à 150 000.

10. Composition selon l'une des revendications précédentes, **caractérisée en ce que** le copolymère est présent en une teneur se situant dans la plage allant de 0,1 % à 30 % en poids, par rapport au poids total de la composition, de préférence se situant dans la plage allant de 0,1 % à 25 % en poids.

11. Composition selon l'une des revendications précédentes, **caractérisée en ce que** l'huile d'hydrocarbures non polaires ayant de 8 à 16 atomes de carbone est choisie parmi les alcanes en C₈-C₁₆ ramifiés et les alcanes en C₈-C₁₆ linéaires.

12. Composition selon l'une des revendications 1 à 10, **caractérisée en ce que** l'huile d'esters est choisie parmi les triglycérides constitués d'esters d'acides gras en C₄-C₂₄ et de glycérol ; les huiles de monoesters de formule R₄COOR₅ dans laquelle R₄ représente une chaîne d'hydrocarbure comprenant de 5 à 19 atomes de carbone et R₅ représente une chaîne d'hydrocarbure, en particulier une chaîne d'hydrocarbure ramifié, contenant de 4 à 20 atomes de carbone, à condition que R₄ + R₅ soit ≥ 9 atomes de carbone ; les esters d'acide lactique et d'alcool en C₁₀₋₂₀ ; les diesters d'acide malique et d'alcool en C₁₀-C₂₀ ; les esters de pentaérythritol et d'acide carboxylique en C₈-C₂₂ ; les diesters de formule (II) R6-O-C(=O)-R'6-C(=O)-O-R"6, R6 et R"6, qui sont identiques ou différents, représentant une chaîne alkyle en C₄ à C₁₂ saturée ou insaturée et linéaire ou ramifiée présentant éventuellement au moins un cycle saturé ou insaturé et R'6 représentant une chaîne alkylène en C₁ à C₄ saturée ou insaturée ; les diesters de formule (III) R7-C(=O)-O-R'7-O-C(=O)-R"7, R7 et R"7, qui sont identiques ou différents, représentant une chaîne alkyle en C₄ à C₁₂ saturée ou insaturée et linéaire ou ramifiée et R'7 représentant une chaîne alkylène en C₁ à C₄ saturée ou insaturée.

13. Composition selon l'une des revendications 1 à 10, **caractérisée en ce que** les huiles de carbonates sont choisies parmi les carbonates de formule R8-O-CO-O-R9, R8 et R9, qui sont identiques ou différents, représentant une chaîne alkyle en C₄ à C₁₂.

14. Composition selon l'une des revendications 1 à 10, **caractérisée en ce qu'**elle comprend un monoalcool de 2 à 6 atomes de carbone de préférence choisi parmi l'éthanol ou le butan-1-ol et préférentiellement l'éthanol.

15. Composition selon l'une des revendications précédentes, **caractérisée en ce qu'**elle comprend un milieu physiologiquement acceptable.

16. Procédé cosmétique non thérapeutique pour le traitement de substances kératiniques, comprenant l'application, sur les substances kératiniques, d'une composition selon l'une des revendications précédentes.
